# EUROPÄISCHE PATENTSCHRIFT

(11) **EP 1 604 992 B1**
(45) Veröffentlichungstag und Bekanntmachung des Hinweises auf die Patenterteilung: **05.08.2009**
(21) Anmeldenummer: 05011046.9
(22) Anmeldetag: 21.05.2005
(51) Int. Cl.: C07D 493/18

(54) **Verfahren zur Herstellung von 10alpha-[4'-(S-S-Dioxothothiomorpholin-1'-yl)]-10-Deoxo-10-Dihydroartemisinin**
Process for the preparation of 10alpha-[4'-(S-S-Dioxothothiomorpholin-1'-yl)]-10-Deoxo-10-Dihydroartemisinin
Procédé pour la préparation de 10alpha-[4'-(S-S-Dioxothothiomorpholin-1'-yl)] -10-Deoxo-10-Dihydroartemisinine

(30) Priorität: 08.06.2004 DE 102004027871
(43) Veröffentlichungstag der Anmeldung: 14.12.2005
(73) Patentinhaber: Saltigo GmbH, 40764 Langenfeld (DE)
(72) Erfinder: Hölzer, Bettina, 51379 Leverkusen (DE)
(74) Vertreter: Wichmann, Birgid

(56) Entgegenhaltungen:
- WO-A-00/04026
- JOHNSON J L; WERBEL L M: "Synthesis and antileishmanial activity of 6-methoxy-4-methyl-N-[6-(substituted-1-pip erazinyl)hexyl]-8-quinolina mines and related compounds" JOURNAL OF MEDICINAL CHEMISTRY, Bd. 26, Nr. 2, 1983, Seiten 185-194, XP002347850

## Beschreibung

Die Erfindung betrifft ein neues, verbessertes Verfahren zur Herstellung von 10α-[4'-(S,S-dioxothiomorpholin-1'-yl)]-10-deoxo-10-dihydroartemisinin.

Aus der WO-A-03/076446 sind eine Vielzahl C-10 substituierter Derivate des Dihydroartemisinins der nachfolgenden Formel bekannt, 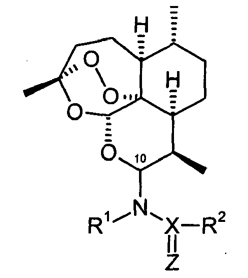 worin R¹ und R² verschiedenste organische, ggf. Heteroatom-Gruppierungen enthaltende Reste darstellen können, X z.B. für S, eine S(=O), PR³, P-O-R³ oder P-N(R⁴)-R³ Gruppe mit R³ und R⁴ als wiederum organischen Resten steht und Z für Sauerstoff, Schwefel oder einen Rest NR⁵ steht, mit R⁵ als wiederum organischem Rest.

Aus der WO-A-00/04024 sind weitere C-10 substituierte Derivate des Dihydroartemisinins der allgemeinen Formel (1) bekannt, 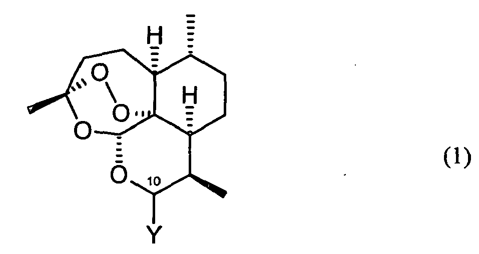 wobei Y für ein Halogen-Atom, einen gegebenenfalls substituierten Cycloalkyl-, Aryl-, einen über ein C-Atom verknüpften Heteroaryl- oder einen Heterocycloalkyl-Rest steht oder für eine Gruppe NR¹R², wobei R¹ für Wasserstoff oder einen gegebenenfalls substituierten Alkyl-, Alkenyl- oder Alkinyl-Rest steht und R² für einen gegebenenfalls substituierten Alkyl-, Alkenyl-, Alkinyl-, Cycloalkyl-, Aryl- oder Aralkyl-Rest, oder aber R¹ und R² zusammen mit dem angrenzenden Stickstoff-Atom eine gegebenenfalls substituierte heterocyclische Gruppe darstellen oder eine Amino-Gruppe abgeleitet von einem gegebenenfalls substituierten Aminosäureester.

Derartige in der WO-A-03/076446 und WO-A-00/04024 beschriebene Verbindungen sind wirksam bei der Behandlung parasitärer Infektionen wie Malaria, Neosporose oder Kozidiose.

Genannt wird in der WO-A-00/04024 auch das 10α-[4'-(S,S-dioxothiomorpholin-1'-yl)]-10-deoxo-10-dihydro-artemisinin (1a) als Verbindung der allgemeinen Formel (1).

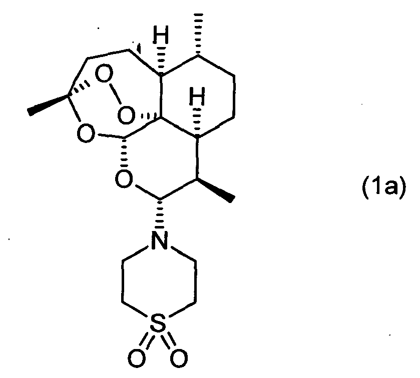

In WO-A-00/04024 wird die Synthese der Verbindungen gemäß der allgemeinen Formel (1) beschrieben durch Umsetzung eines Dihydroartemisinins der Formel (2) 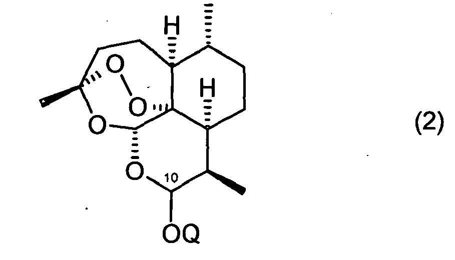 wobei Q ein Wasserstoff-Atom oder eine Trimethylsilylgruppe bedeutet, mit einem Halogenierungsmittel unter Bildung einer Verbindung der Formel (1), bei der Y für ein Halogen-Atom steht, und nachfolgende Umsetzung mit einem Grignard der Formel YMgX mit X = Halogen oder mit einem Amin der allgemeinen Formel HNR¹R² .

Die Herstellung von 10α-[4'-(S,S-dioxothiomorpholin-1'-yl)]-10-deoxo-10-dihydro-artemisinin (1a) wird in der WO-A-00/04024 ausgehend von Dihydroartemisinin der Formel (2a) und Thiomorpholin der Formel (3) beschrieben. Gemäß den Beispielen 3(a), 3(b), 6 und 7 wird zunächst Dihydroartemisinin (2a) mit Chlor- und dann mit Bromtrimethylsilan umgesetzt und danach werden 3 Äquivalente Thiomorpholin zugegeben unter Bildung der Verbindung der Formel (4). Diese Verbindung der Formel (4) wird als Zwischenprodukt isoliert. Beide Umsetzungen erfolgen bei sehr niedrigen Temperaturen im Bereich von 0°C und Raumtemperatur. Die nachfolgende Oxidation der Verbindung der Formel (4) liefert die Zielverbindung der Formel (1a), wobei NMO 4-Methylmorpholin-N-oxid und TPAP = Tetrapropylammonium perruthenat bedeutet

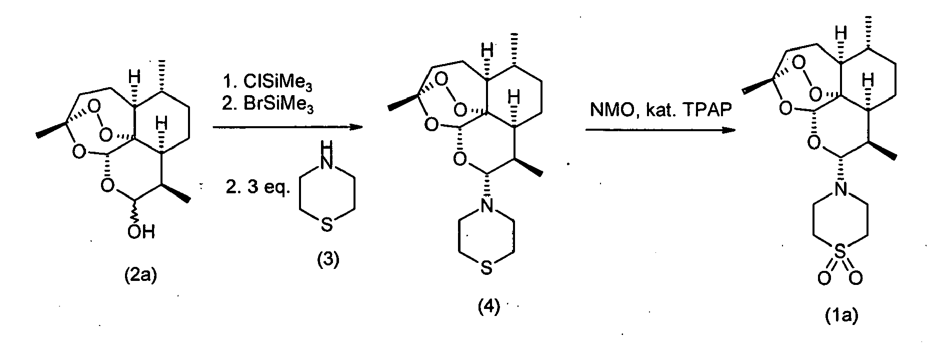

Nachteilig an dieser Synthese ist, dass es sich um einen zweistufigen Prozess handelt und 3 Äquivalente des teuren Einsatzstoffes Thiomorpholin eingesetzt werden müssen.

Es war somit Aufgabe der vorliegenden Erfindung, ein verbessertes Verfahren zur Herstellung von 10α-[4'-(S,S-dioxothiomorpholin-1'-yl)]-10-deoxo-10-dihydro-artemisinin (1a) bereitzustellen.

Überraschenderweise ist es möglich, 10α-[4'-(S,S-dioxothiomorpholin-1'-yl)]-10-deoxo-10-dihydro-artemisinin (1a) in einem einstufigen Verfahren aus 10-Halogeno-10-deoxo-10-dihydroartemisinin (1b) und Thiomorpholinoxid (3) herzustellen.

Gegenstand der Erfindung ist ein Verfahren zur Herstellung von 10α-[4'-(S,S-dioxothiomorpholin-1'-yl)]-10-deoxo-10-dihydro-artemisinin (1a), welches dadurch gekennzeichnet ist, dass man eine Verbindung der Formel (2) 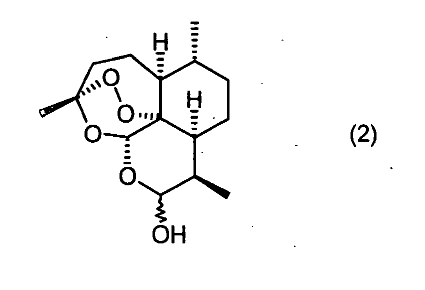 mit HX, wobei X für Brom, Chlor oder Iod steht, zu Verbindungen der Formel (1b) umsetzt, 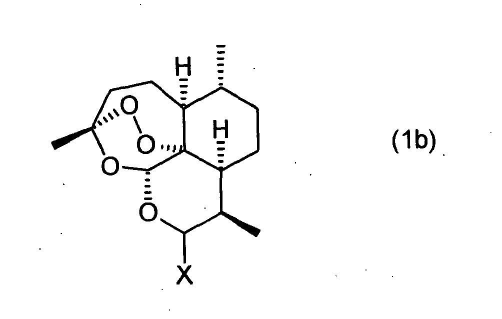 wobei X die vorgenannte Bedeutung besitzt, und die Verbindung der Formel (1b) mit Thiomorpholindioxid der Formel (3) 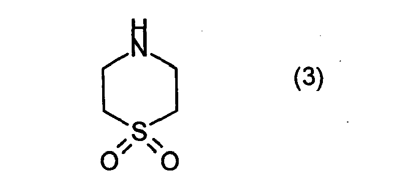 bei einer Temperatur im Bereich von -30°C bis +20 °C umsetzt.

Die erfolgreiche Durchführbarkeit des Verfahrers ist in mehrfacher Hinsicht überraschend:

Bekannt ist, dass Thiomorpholindioxid aufgrund des elektronenziehenden Effekts der Sulfongruppe ein deutlich schlechteres Nucleophil als Thiomorpholin ist. Somit sind für nucleophile Substitutionsreaktionen unter Verwendung von Thiomorpholindioxid deutlich höhere Reaktionstemperaturen notwendig als bei Einsatz von Thiomorpholin (siehe z.B. J. L. Johnson, L. M. Werbel, J. Med. Chem. 1983, 26, 185-194). Gleichzeitig ist aber aus der Literatur hinlänglich bekannt, dass sich Dihydroartemisinin der Formel (2) und strukturell verwandte Verbindungen bei Erwärmung aufgrund der thermischen Instabilität der Peroxogruppe zersetzen (siehe z.B. A. J. Lin, A. D. Theohanrides, D. L. Klayman, Tetrahedron 1986, 42 (8), 2181-4; X. D. Luo, H. J. C. Yeh, A. Brossi, J. L. Flippen-Anderson, R. Gilardi, Heterocycles 1985, 23 (4), 881-7). Die Abtrennung dabei gebildeter Zerfallsprodukte vom gewünschten Produkt ist aufgrund der strukturellen Ähnlichkeit schwierig. Daher sollten hohe Reaktionstemperaturen vermieden werden, um die Bildung von Zerfallsprodukten zu vermeiden. Dies würde längere Reaktionszeiten bedingen.

Bei längeren Reaktionszeiten bei niedrigen Temperaturen ist aber mit der vermehrten Bildung von Nebenprodukten wie den Verbindungen der Formel (5) und (6) zu rechnen, die bereits in der Literatur beschrieben sind (siehe R. K. Haynes, H. Chan, M. Cheung, S. T. Chung, W. Lam, H. Tsang, A. Voerste, I. D. Williams, Eur. J. Org. Chem 2003, 2098-2114).

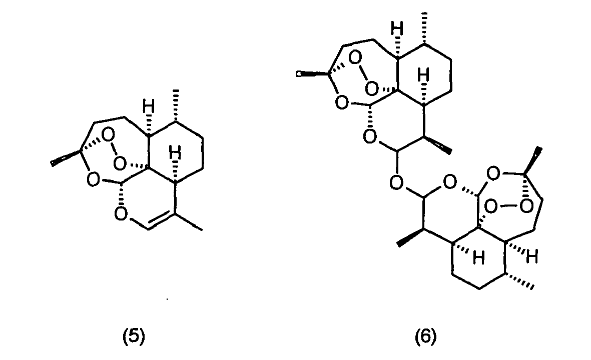

Es ist somit überraschend, dass die Reaktion des Dihydroartemisinin-Derivates der Formel (1b) mit Thiomorpholindioxid der Formel (3) bei niedrigen Temperaturen ohne signifikante Bildung von Nebenprodukten stattfindet.

Thiomorpholindioxid ist ein käuflich erhältliches Reagenz.

Im erfindungsgemäßen Verfahren wird als Edukt eine Verbindung der Formel (2) eingesetzt.

Diese Verbindung der Formel (1b) kann gemäß WO-A-00/04024 zuvor *in situ* gebildet werden und muss nicht isoliert werden. Zwecks Herstellung kann man Dihydroartemisinin der Formel (2) mit einem Halogenierungsmittel umsetzen. Geeignete Halogenierungsmittel sind HCl, HBr oder HI.

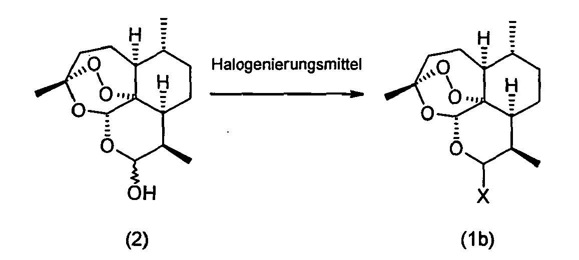

Bevorzugterweise wird Dihydroartemisinin der Formel (2) mit HBr-Gas oder besonders bevorzugt HCl-Gas in Gegenwart von CaCl₂ umgesetzt. HBr- bzw. HCl-Gas sind kostengünstige und leicht verfügbare Reagenzien.

Zur Verbesserung der Ausbeute und Vermeidung von Nebenprodukten ist es vorteilhaft, das bei dieser Reaktion entstehende Wasser zu entfernen. Dies erfolgt geeigneterweise durch Einsatz von Molekularsieb oder Trocknungsmitteln wie MgCl₂, CaCl₂, LiCl, Na₂SO₄, MgSO₄ oder CaSO₄. Es hat sich bewährt, 1-20 Äquivalente, bevorzugt 1-5 Äquivalente, besonders bevorzugt 2-3 Äquivalente des Trocknungsmittels einzusetzen.

Die in-situ Bildung von Verbindung (1b) wird bei einer Temperatur im Bereich von -30°C bis +20 °C, bevorzugt im Bereich von -20°C bis + 10 °C und insbesondere im Bereich von -10°C bis 0 °C durchgeführt.

Es hat sich bewährt, diese Reaktion in polaren, nicht-nukleophilen Lösungsmitteln, bevorzugt in halogenierten aliphatischen oder aromatischen Kohlenwasserstoffen, insbesondere chlorierten aliphatischen oder aromatischen Kohlenwasserstoffen durchzuführen. Einsetzbar ist z.B. Chlorbenzol. Besonders bevorzugt ist Methylenchlorid.

Bei der erfindungsgemäßen Umsetzung der in-situ gebildeten Verbindung der Formel (1b) mit Thiomorpholindioxid werden üblicherweise 1-3 Äquivalente Thiomorpholindioxid eingesetzt. Vorteilhafterweise läuft die Reaktion jedoch auch mit deutlich geringeren Mengen als 3 Äquivalenten Thiomorpholin ab. Bevorzugt werden 1 - 2,5, besonders bevorzugt 1 - 2,2 und insbesondere 2 Äquivalente Thiomorpholindioxid verwendet. Dies führt zu einer optimalen Unterdrückung von Nebenreaktionen.

Die erfindungsgemäße Umsetzung der Verbindung der Formel (1b) wird bei einer Temperatur im Bereich von -30°C bis +20 °C, bevorzugt im Bereich von -20°C bis + 10 °C und insbesondere im Bereich von -10°C bis 0 °C durchgeführt.

Üblicherweise wird diese Umsetzung in dem gleichen Lösungsmittel durchgeführt wie die in-situ Herstellung des Ausgangsmaterials der Formel (1b).

Es hat sich bewährt, in Gegenwart einer Hilfsbase zu arbeiten, die stärker basisch als Thiomorpholindioxid ist, um das bei der Reaktion der Verbindung der Formel (1b) mit Thiomorpholindioxid entstehenden HX mit X = Halogen, bevorzugt Chlor oder Brom, abzufangen. Diese Maßnahme erlaubt eine Minimierung der Menge an Thiomorpholindioxid. Üblicherweise wird 1 Äquivalent der Hilfsbase eingesetzt. Bevorzugt werden Amine wie Pyridin oder Triethylamin verwendet. Besonders bevorzugt ist Triethylamin. Überraschenderweise wird gefunden, dass es bei Verwendung dieser Hilfsbase nicht zur einer vermehrten Bildung des Eliminierungsproduktes der Formel (5) kommt.

Die Durchführung des erfindungsgemäßen Verfahrens erfolgt üblicherweise so, dass zu der Lösung der in-situ erzeugten Verbindung der allgemeinen Formel (1b) bei der zuvor angegebenen Reaktionstemperatur eine Lösung des Thiomorpholindioxids sowie der Hilfsbase in einem Lösungsmittel zugegeben wird, welches bevorzugt identisch mit dem Lösungsmittel ist, in dem die Verbindung (1b) gelöst ist. Nach geeignetem Nachrühren erfolgt eine Phasentrennung. Aus der organischen Phase kann durch übliche Aufarbeitungsmethoden wie Destillation und Umkristallisation das gewünschte Produkt 10α-[4'-(S,S-dioxothiomorpholin-1'-yl)]-10-deoxo-10-dihydro-artemisinin (1a) gewonnen werden. Die Umkristallisation wird bevorzugt aus einem Alkohol, besonders bevorzugt aus Methanol, Ethanol, Butanol oder insbesondere i-Propanol durchgeführt.

Über das erfindungsgemäße Verfahren sind auch Salze des 10α-[4'-(S,S-dioxothiomorpholin-1'-yl)]-10-deoxo-10-dihydro-artemisinins (1a) zugänglich. Dies schließt jegliche Salze ein, die durch Reaktion der Verbindung (1a) mit einer geeigneten organischen oder anorganischen Säure erhalten werden können. Bevorzugte Salze werden durch Umsetzung mit einer Mineralsäure wie HCl oder HBr unter Protonierung des Stickstoffs erhalten.

### Beispiel 1:

### Herstellung von 10α-[4'-(S,S-dioxothiomorpholin-1'-yl)]-10-deoxo-10-dihydro-artemisinin (1a)

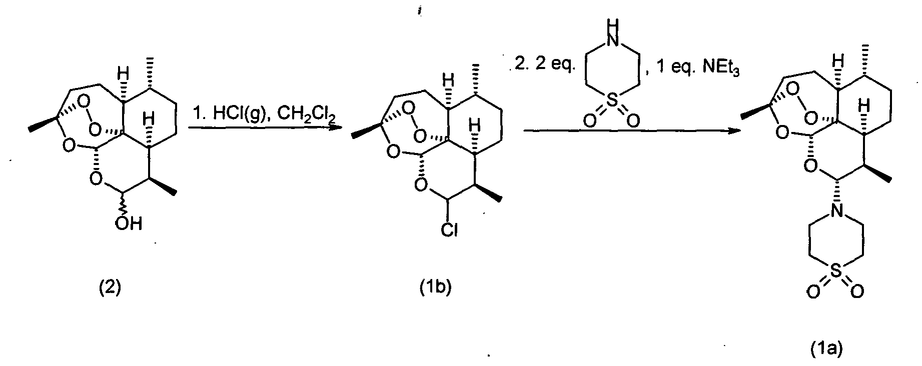

In eine Suspension aus
60 g Verbindung der Formel (1b) (mit einem Gehalt von 98,4 Gew.% entsprechend 211 mmol) und
37.7 g CaCl₂-Pulver (366 mmol) in
800 ml CH₂Cl₂ werden bei -10 °C in 70 min
ca. 26 g HCl-Gas (722 mmol) eingeleitet.

Die Reaktion verläuft anfangs exotherm, so dass die Innentemperatur auf -4 °C bei einer Mantelkühlung von -10 °C ansteigt. Es wird solange HCl-Gas eingeleitet, bis ein Durchschlag am Blasenzähler zu erkennen ist. Man lässt 15 min nachrühren und leitet anschließend 85 min ein Stickstoff-Strom durch das Reaktionsgefäß, um das überschüssige HCl-Gas zu verdrängen.

Anschließend werden in 50 min bei einer Temperatur von -5 bis -10 °C eine Lösung von 56.9 g Thiomorpholindioxid (mit einem Gehalt von 98 Gew.% entsprechend 422 mmol) und 23.5 g Triethylamin (mit einem Gehalt von 98 Gew.% entsprechend 232 mmol) in 200 ml CH₂Cl₂ zugegeben.

Nach 2 h Nachrühren bei einer Temperatur von -5 °C werden bei +6 °C 500 ml Wasser zugegeben. Nach Erwärmen auf RT wird die organische Phase (unten) abgetrennt und die wässrige Phase zweimal mit je 100 ml CH₂Cl₂ extrahiert. Das Lösungsmittel der vereinigten organischen Phasen wird unter vermindertem Druck entfernt. Anschließend werden 435 g iso-Propanol zugeben und der Rückstand bei 50 °C gelöst. Man kühlt in einer Stunde auf 20°C ab und rührt 3 Stunden bei dieser Temperatur nach. Der Feststoff wird anschließend abfiltriert (33.1 g) und erneut in 248.4 g iso-Propanol bei 60 °C gelöst. Nach Abkühlen auf 20°C innerhalb von 1 Stunde und 3 Stunden Nachrühren bei 20°C wird der farblose Feststoff abfiltriert. Es werden 27.4 g (32.1 %) 10α-[4'-(S,S-dioxothiomorpholin-1'-yl)]-10-deoxo-10-dihydro-artemisinin (Artemisone) erhalten (100 Gew% laut HPLC-Analyse).

**¹H-NMR (400 MHz, CDCl₃):** δ = 5.26 (s, 1 H), 4.20 (d, J = 10.3 Hz, 1 H), 3.46-3.18 (m, 8 H), 2.62-2.54 (m, 1 H), 2.36-2.28 (m, 1 H), 2.02-1.20 (m, 9 H), 1.35 (s, 3 H), 1.06-0.92 (m, 1 H), 0.93 (d, J = 6.0 Hz, 3 H), 0.78 (d, J = 7.1 Hz, 3 H).

## Patentansprüche

1. Verfahren zur Herstellung von 10α-[4'-(S,S-dioxothiomorpholin-1'-yl)]-10-deoxo-10-dihydroartemisinin (1a), **dadurch gekennzeichnet, dass** man eine Verbindung der Formel (2) 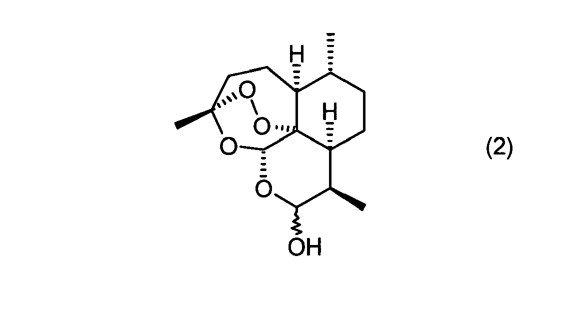 mit HX, wobei X für Brom, Chlor oder Iod steht, zu Verbindungen der Formel (1b) umsetzt, 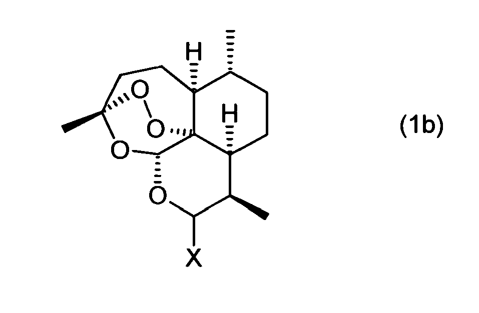 wobei X die vorgenannte Bedeutung besitzt, und die Verbindung der Formel (1b)
mit Thiomorpholindioxid der Formel (3) 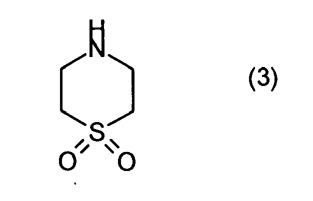 bei einer Temperatur im Bereich von -30°C bis +20 °C umsetzt.

2. Verfahren nach Anspruch 1, **dadurch gekennzeichnet, dass** eine Verbindung der Formel (1b) eingesetzt wird, wobei X für Brom oder Chlor steht.

3. Verfahren nach Anspruch 1 oder 2, **dadurch gekennzeichnet, dass** die Verbindung der Formel (1b) *in situ* gebildet und nicht isoliert wird vor der Umsetzung gemäß Anspruch 1 oder 2.

4. Verfahren nach einem oder mehreren der Ansprüche 1 bis 2, **dadurch gekennzeichnet, dass** die Umsetzung mit gasförmigem HX, wobei X für Brom oder Chlor steht, durchgeführt wird.

5. Verfahren nach einem oder mehreren der Ansprüche 1 bis 4, **dadurch gekennzeichnet, dass** die Umsetzung der Verbindung der Formel (2) mit gasförmigem HX in Gegenwart von Molekularsieb oder einem Trocknungsmittel, bevorzugt MgCl₂, CaCl₂, LiCl, Na₂SO₄, MgSO₄ oder CaSO₄ durchgeführt wird.

6. Verfahren nach nach einem oder mehreren der Ansprüche 1 bis 5, **dadurch gekennzeichnet, dass** die Umsetzung der Verbindung der Formel (2) bei einer Temperatur im Bereich von -10°C bis 0 °C durchgeführt wird.

7. Verfahren nach einem oder mehreren der Ansprüche 1 bis 6, **dadurch gekennzeichnet, dass** 1 bis 2 Äquivalente Thiomorpholindioxid eingesetzt werden.

8. Verfahren nach einem oder mehreren der Ansprüche 1 bis 7, **dadurch gekennzeichnet, dass** die Umsetzung der Verbindung der Formel (1b) mit Thiomorpholindioxid der Formel (3) in Gegenwart einer Hilfsbase, die stärker basisch ist als Thiomorpholindioxid ist durchgeführt wird.

## Claims

1. Process for preparing 10α-[4'-(S,S-dioxothiomorpholin-1'-yl)]-10-deoxo-10-dihydroartemisinin (1a), **characterized in that** a compound of the formula (2) 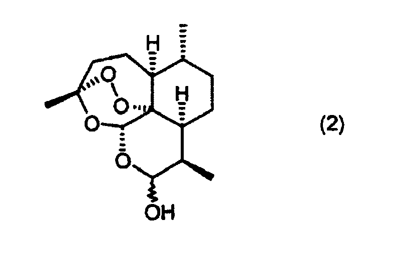 is reacted with HX in which X is bromine, chlorine or iodine to give compounds of the formula (1b) 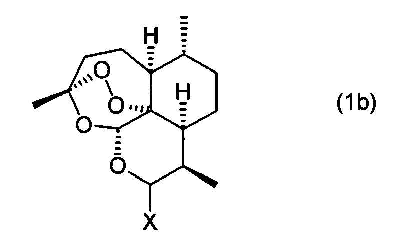 in which X is as defined above, and the compound of the formula (1b)
is reacted with thiomorpholine dioxide of the formula (3) 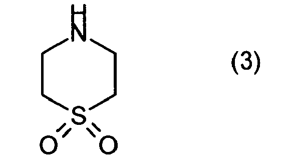 at a temperature in the range of -30°C to +20°C.

2. Process according to Claim 1, **characterized in that** a compound of the formula (1b) in which X is chlorine or bromine is used.

3. Process according to Claim 1 or 2, **characterized in that** the compound of the formula (1b) is formed *in situ* and is not isolated before the reaction according to Claim 1 or 2.

4. Process according to one or more of Claims 1 to 2, **characterized in that** the reaction is carried out with gaseous HX in which X is bromine or chlorine.

5. Process according to one or more of Claims 1 to 4, **characterized in that** the reaction of the compound of the formula (2) with gaseous HX is carried out in the presence of molecular sieve or a desiccant, preferably MgCl₂, CaCl₂, LiCl, Na₂SO₄, MgSO₄ or CaSO₄.

6. Process according to one or more of Claims 1 to 5, **characterized in that** the reaction of the compound of the formula (2) is carried out at a temperature in the range of -10°C to 0°C.

7. Process according to one or more of Claims 1 to 6, **characterized in that** 1 to 2 equivalents of thiomorpholine dioxide are used.

8. Process according to one or more of Claims 1 to 7, **characterized in that** the reaction of the compound of the formula (1b) with thiomorpholine dioxide of the formula (3) is carried out in the presence of an auxiliary base which is more strongly basic than thiomorpholine dioxide.

## Revendications

1. Procédé de fabrication de 10α-[4'-(S,S-dioxothiomorpholin-1'-yl)]-10-désoxo-10-dihydroartémisinine (1a), **caractérisé en ce qu'**un composé de formule (2) 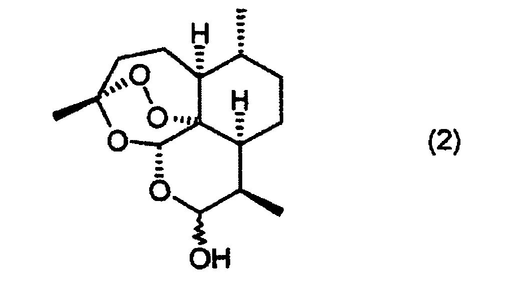 est mis en réaction avec HX, X représentant le brome, le chlore ou l'iode, pour former des composés de formule (1b) 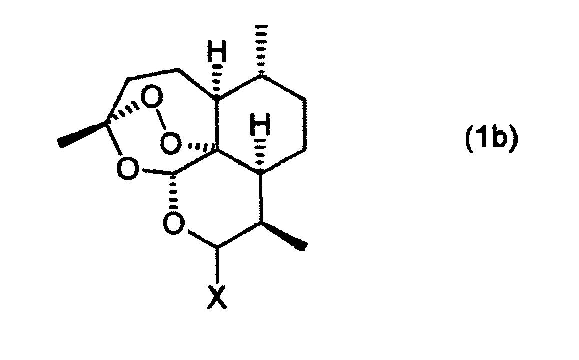 X ayant la signification susmentionnée, et le composé de formule (1b) est mis en réaction avec du dioxyde de thiomorpholine de formule (3) 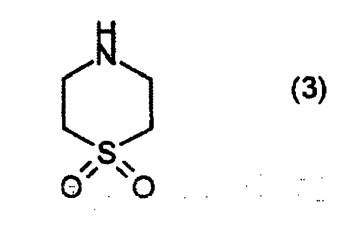 à une température dans la plage allant de -30 °C à +20 °C.

2. Procédé selon la revendication 1, **caractérisé en ce qu'**un composé de formule (1b), dans laquelle X représente le brome ou le chlore, est utilisé.

3. Procédé selon la revendication 1 ou 2, **caractérisé en ce que** le composé de formule (1b) est formé in situ et n'est pas isolé avant la réaction selon la revendication 1 ou 2.

4. Procédé selon une ou plusieurs des revendications 1 à 2, **caractérisé en ce que** la réaction est réalisée avec HX gazeux, X représentant le brome ou le chlore.

5. Procédé selon une ou plusieurs des revendications 1 à 4, **caractérisé en ce que** la réaction du composé de formule (2) avec HX gazeux est réalisée en présence d'un tamis moléculaire ou d'un agent dessicatif, de préférence MgCl₂, CaCl₂, LiCl, Na₂SO₄, MgSO₄ ou CaSO₄.

6. Procédé selon une ou plusieurs des revendications 1 à 5, **caractérisé en ce que** la réaction du composé de formule (2) est réalisée à une température dans la plage allant de -10 °C à 0 °C.

7. Procédé selon une ou plusieurs des revendications 1 à 6, **caractérisé en ce qu'**un à deux équivalents de dioxyde de thiomorpholine sont utilisés.

8. Procédé selon une ou plusieurs des revendications 1 à 7, **caractérisé en ce que** la réaction du composé de formule (1b) avec le dioxyde de thiomorpholine de formule (3) est réalisée en présence d'une base auxiliaire qui est plus fortement basique que le dioxyde de thiomorpholine.
